(19) Europäisches Patentamt
European Patent Office
Office européen des brevets

(11) Veröffentlichungsnummer: **0 039 893**
**B1**

(12) # EUROPÄISCHE PATENTSCHRIFT

(45) Veröffentlichungstag der Patentschrift: **18.07.84**

(51) Int. Cl.³: **C 07 J 7/00**, C 07 J 9/00

(21) Anmeldenummer: **81103422.2**

(22) Anmeldetag: **06.05.81**

(54) **Verfahren zum Abbau der C-20-Carboxylgruppe in delta-4- und gegebenenfalls weitere Doppelbindungen enthaltenden BNC-Verbindungen sowie neue C-21-Steroide.**

(30) Priorität: **12.05.80 AT 2537/80**
**12.05.80 AT 2538/80**

(43) Veröffentlichungstag der Anmeldung:
**18.11.81 Patentblatt 81/46**

(45) Bekanntmachung des Hinweises auf die Patenterteilung:
**18.07.84 Patentblatt 84/29**

(84) Benannte Vertragsstaaten:
**BE CH DE FR LI NL**

(56) Entgegenhaltungen:
**US - A - 2 492 192**

**JOURNAL OF ORGANIC CHEMISTRY, Band 30, Nr. 11, November 1965, Seiten 3760-3761 Washington D.C., U.S.A. D.B. DENNEY et al.: "Degradation of acids to alcohols by the carboxy-inversion reaction"**
**JOURNAL OF ORGANIC CHEMISTRY, Band 30, Nr. 17, 24. August 1973, Seiten 3040-3046 Washington D.C., U.S.A. P. ROSEN et al.: "15-Oxa steroids"**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Preuss, Wolfgang, Dr., Finkenweg 12, D-4019 Monheim (DE)**

**EP 0 039 893 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der offengelegten Europäischen Patentanmeldung 79 101 036.6 (004 913) ist u. a. ein Verfahren zur Herstellung von 17-C-Steroid-α-propionsäureverbindungen durch mikrobiellen Seitenkettenabbau an 17-C-Seitenkettensteroidsubstraten. Auf diesem Wege gelingt insbesondere die Herstellung von 3-Oxo-pregna-4-en-20-carbonsäure (Δ4-BNC) und/oder 3-Oxopregna-1,4-dien-20-carbonsäure (Δ1,4-BNC). Bei diesem Verfahren wird mit Mikroorganismen Defektmutanten gearbeitet, die auch in Abwesenheit von den Steroidringabbau und/oder das Wachstum hemmenden Inhibitoren Steroidverbindungen mit den 17-C-α-Propionsäurerest liefern. Diese Defektmutanten werden gemäß den Angaben der genannten Europäischen Patentanmeldung gewonnen und gezüchtet. Eine Abwandlung dieses Verfahrens wird in der älteren Europäischen Patentanmeldung 79 104 165.0 (0 015 308) geschildert.

Die Strukturformeln der Δ4-BNC und Δ1,4-BNC sind die folgenden:

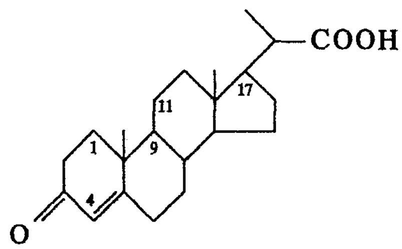

(Δ4-BNC)

3-Oxo-pregna-4-en-20-carbonsäure.

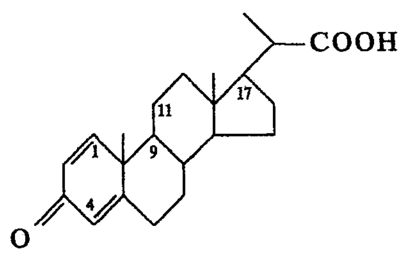

(Δ1,4-BNC)

3-Oxo-pregna-1,4-dien-20-carbonsäure.

In der DE-OS 2 839 033 sind strukturanaloge Steroid-20-Carbonsäuren mit einer zusätzlichen Doppelbindung in 9(11)-Stellung sowie Verfahren zu ihrer Herstellung erwähnt. Geschildert ist dort insbesondere eine Möglichkeit zur Herstellung der Δ1,4,9(11)-BNC. Die US-PS 4 062 880 beschreibt die strukturverwandte Δ4, 9(11)-BNC.

Die bisher erwähnten BNC-Verbindungen tragen nur in Position 3 eine funktionelle Gruppe im Ringsystem. Alle pharmakologisch wirksamen Corticosteroide enthalten jedoch zusätzliche Sauerstoff-Funktionen. Besonders wichtig sind dabei u. a. die Positionen 11, 17 und 21. Üblicherweise werden einige dieser Sauerstoff-Funktionen chemisch eingeführt, dazu gehören insbesondere die Positionen 17 und 21.

Die Oxidation der Position 11 in Steroidverbindungen erfolgt dagegen bevorzugt mikrobiell. In der Fachliteratur ist eine Vielzahl mikrobieller Steroid-Oxidationen in Position 11 beschrieben. Verwiesen wird in diesem Zusammenhang auf die folgenden Literaturreferate sowie die dort genannten Originalveröffentlichungen:

F. Drawert »Biosynthese von Hydroxy-Verbindungen«; Houben-Weyl »Methoden der organischen Chemie« (1978) 6/1d, Seiten 378—388;
T.H. Stoudt, Adv. Appl. Microbiol. 2 (1960), Seiten 190—195, sowie
Handbuch W.Charney und H.L. Herzog »Microbial Transformations of Steroids« Academic Press (1967) New York, Seite 29.

Beschrieben werden hier mit zahlreichen Verweisungen auf bestimmte Mikroorganismenstämme, insbesondere aus der Klasse de. Fungi, die mikrobielle 11-Hydroxylierung verschiedenartiger Steroidverbindungen und die dabei entstehenden Syntheseprodukte.

Für eine hohe pharmakologische Wirksamkeit ist im allgemeinen die Konfiguration 11 β-Hydroxyl oder 11-Oxo erwünscht. Um in Position 11 β-hydroxylierte Steroide zu erhalten, werden entweder Mikroorganismenstämme verwendet, die eine derartige Hydroxylgruppe stereoselektiv einführen, oder es werden andere Mikroorganismen verwendet, die überwiegend oder ausschließlich stereoselektiv in 11 α-Position hydroxylieren. In diesem Fall wird zur Gewinnung der 11 β-hydroxylierten Steroide zunächst chemisch zum 11-Keton oxydiert, und anschließend mit einem geeigneten Reduktionsmittel reduziert, wobei stereoselektiv die 11 β-Hydroxyverbindung gebildet werden kann. Zur einschlägigen Literatur dieser anschließenden chemischen Umwandlung wird beispielsweise verwiesen auf

L. Fieser, M. Fieser »Steroide« Verlag Chemie (Weinheim 1961) Seiten 737 ff. sowie die dort zitierte Originalliteraturstelle J. Am. Chem. Soc. 77, 4436 (1955).

Die Herstellung von Δ4- und/oder Δ1,4-BNC-Verbindungen mit einer Sauerstoff-Funktion in 11-Stellung ist beispielsweise in der DE-OS 2 839 033 beschrieben. Nach diesem Verfahren werden die in 11-Stellung unsubstituierten BNC-Verbindungen in einem wäßrigen Nährmedium unter aeroben Bedingungen auf mikrobiologischem Wege in 11-Stellung oxidiert. Insbesondere wird die Herstellung der 11 α-Hydroxy- oder 11-β-Hydroxy-1,4-BNC und der 11-Keto-1,4-BNC beschrieben.

In der älteren Europäischen Patentanmeldung 81 100 145.2 (34 248) wird unter anderem das bis dahin nicht beschriebene Säurechlorid der Δ1,4-BNC sowie ein Verfahren zu seiner Herstellung beschrieben. Das Pregna-1,4-dien-3-on-20-carbonylchlorid (Δ1,4-BNC-Chlorid), analoge andere Säurehalogenide der Δ1,4-BNC sowie entsprechende Säurehalogenide der Δ4-BNC können durch Umsetzung der freien Säuren mit Halogenierungsmitteln erhalten werden. Vorzugsweise wird unter höchstens mäßig erhöhten Temperaturen gearbeitet, insbesondere bei Temperaturen nicht über 15°C und insbesondere bei Temperaturen unter 5°C. Es wird weiterhin bevorzugt, das Halogenierungsmittel in annähernd stöchiometrischer Menge oder mit nur einem begrenzten molaren Überschuß (bis zu etwa 10%) einzusetzen. Zweckmäßig wird weiterhin in einem inerten Lösungsmittel, beispielsweise in halogenierten Kohlenwasserstoffen, gearbeitet. Es gelingt auf diese Weise die selektive Halogenierung der 20-Carboxylgruppe zur entsprechenden Säurechloridgruppierung ohne eine unerwünschte Halogenierung im Ringsystem der Steroidverbindung auszulösen.

Analoge Verfahren zur Herstellung ähnlicher BNC-Halogenide sind Gegenstand paralleler älterer Anmeldungen der Anmelderin. So beschreibt die Europäische Patentanmeldung 81 103 332.3 (40 342) (»Neue Steroid-20-Carbonsäureverbindungen und Verfahren zu ihrer Herstellung«, Priorität Österreich A 2629/80 vom 16. 5. 1980) die Herstellung der Halogenide von Δ4,9(11)-BNC, die auch noch weitere Doppelbindungen, z. B. in 1(2)-Stellung aufweisen kann. Die Europäische Patentanmeldung 81 103 424.8 (39 895) (»Neue Pregnan-20-Carbonsäurederivate und Verfahren zu ihrer Herstellung«, Priorität Österreich A 2535/80 vom 12. 5. 1980) schildert die Herstellung von Halogeniden, insbesondere Chloriden, vergleichbarer BNC-Verbindungen, die in 11-Stellung eine Sauerstoff-Funktion und dabei insbesondere eine Hydroxylgruppe oder eine Keto-Gruppe aufweisen.

Die Erfindung will einen Weg schaffen, auf dem — beispielsweise ausgehend von Δ4-BNC und/oder Δ1,4-BNC, die auch in 11-Stellung eine Sauerstoff-Funktion aufweisen können, — durch chemische Umwandlung des Seitenkettensubstituenten in 17-Stellung in einfacher Weise Zwischenprodukte zugänglich werden, die ihrerseits problemlos in pharmakologisch erwünschte Steroidverbindungen umgewandelt werden können. Aufgabe der Erfindung ist weiterhin die Anwendung einer an sich bekannten Carbonsäure-Abbautechnik auf Verbindungen mit Steroidstruktur der angegebenen Art. Schließlich will die Erfindung neue C-21-Steroidverbindungen zur Verfügung stellen, die sich insbesondere durch eine Sauerstoff-Funktion in 11-Stellung auszeichnen.

Gegenstand der Erfindung ist dementsprechend in einer ersten Ausführungsform ein Verfahren zum Abbau der 20-Carboxylgruppe von Δ4- und gegebenenfalls wenigstens eine weitere Doppelbindung in 1(2)- und/oder 9(11)-Stellung aufweisenden Steroid-20-carbonsäuren der allgemeinen Formel I

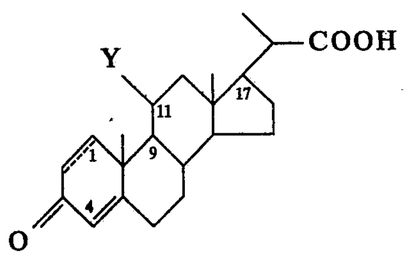

(I)

in der Y Wasserstoff, eine Hydroxylgruppe oder gemeinsam mit dem durch Y substituierten C-Atom eine Carbonylgruppe bedeutet, wobei auch anstelle des Substituenten Y eine 9(11)-en-Bindung vorliegen kann, wobei dieses Verfahren dadurch gekennzeichnet ist, daß man die Steroid-20-carbonsäuren über ihre Säurehalogenide dem Carboxy-Inversions-Abbau durch Umsetzung mit Persäuren und Verseifung des Reaktionsproduktes unterwirft und gewünschtenfalls das Verseifungsprodukt in die gebildeten 20-Hydroxy- und die 20(21)-en-Steroidverbindungen auftrennt.

Erfindungsgemäß kann dieses Gemisch von Reaktionsprodukten beispielsweise durch Umkristallisieren oder chromatografische Trennung in seine Komponenten zerlegt werden.

Die C-20-Alkohole lassen sich durch übliche Reaktionen der organischen Chemie in die entsprechenden Progesteronderivate überführen. Die C-20(21)-Alkene lassen sich nach im Prinzip ebenfalls bekannten Verfahren zu Verbindungen umwandeln, die Sauerstoff-Funktionen an C-20-C-21 haben. Solche Verfahren sind z. B. in L. u. M. Fieser, Steroide, Seiten 677 ff. (Verlag Chemie, Weinheim/Bergstraße, 1961) und der dort zitierten Originalliteratur beschrieben. Die C-20-Alkene werden dabei in Derivate vom Cortexon-Typ umgewandelt.

Der Abbau von Carbonsäuren zu Alkoholen durch Carboxy-Inversions-Reaktion mittels Persäuren und Verseifung des Reaktionsprodukts ist in der Literatur beschrieben, vgl. J. Org. Chem. 30, 3760 (1965), D. B. Denney, N. Sherman »Degradation of Acids to Alcohols by the Carboxy-Inversion Reaction«. In dieser Erstveröffentlichung dieser Verfahrenstechnik wird die Reaktion allerdings nur an einfachen aliphatischen Carbonsäuren geschildert. Die Anwendung dieses Reaktionsweges auf Verbindungen mit einer Steroid-Teilstruktur wird in J. Org. Chem. 38, 3040 (1973), insbesondere Seite 3041/3042 geschildert. Zur Herstellung von Zwischenprodukten zur Synthese von Steroidhormonen (besonders Nebennierenrindenhormonen), die zudem noch Partialstrukturen vom Typ des A-Ringes in $\Delta$4-BNC und $\Delta$1,4-BNC sowie gegebenenfalls weitere reaktive Stellen im Ringgerüst enthalten, ist dieser Abbau jedoch noch nicht angewendet worden. Seine problemlose Anwendbarkeit unter den im folgenden noch im einzelnen geschilderten Verfahrensbedingungen und die dabei erhaltenen Verfahrensergebnisse waren für den Fachmann nicht mit Sicherheit voraussagbar.

Im einzelnen gilt zu den Bedingungen des neuen Verfahrens das folgende:

Die jeweilige BNC-Ausgangsverbindung wird zunächst in das entsprechende 20-Säurehalogenid, insbesondere das Säurechlorid umgewandelt. Hier gelten im einzelnen die Angaben der eingangs erwähnten parallelen Schutzrechtsanmeldungen der Anmelderin. So kann beispielsweise die Darstellung des Säurechlorids aus der freien Steroidcarbonsäure vorteilhaft bei Temperaturen von 0 bis 5°C in Methylenchlorid und mit einem geringen Überschuß von Thionylchlorid als chlorierendem Agens erfolgen. Eine katalytische Menge Pyridin beschleunigt insbesondere bei der $\Delta$4-BNC die Reaktion, ist aber nicht unbedingt erforderlich.

Anstelle von Methylenchlorid können andere inerte Lösungsmittel, z. B. Chloroform oder Ether eingesetzt werden. Ebenso können andere Chlorierungsmittel, z. B. Phosphortrichlorid oder Phosphorpentachlorid verwendet werden, jedoch ist Thionylchlorid vorzuziehen. Das als Katalysator verwendete Pyridin kann auch durch Dimethylformamid ersetzt werden.

Anstelle der genannten Chlorierungsmittel können andere Halogenierungsmittel, z. B. Thionylbromid, Phosphortribromid oder Phosphorpentabromit verwendet werden, welche das entsprechende Säurebromid liefern.

Der Abbau der 20-Carboxylgruppe erfolgt dann mittels der Carboxy-Inversions-Reaktion durch Umsetzung des Säurehalogenids mit einer Persäure und anschließender Verseifung des aus dem gemischten Persäureanhydrid gebildeten Umlagerungsproduktes, siehe hierzu die bereits zitierte Veröffentlichung in J. Org. Chemistry 30, 3760 (1965) D. B. Denney, N. Sherman Degradation of Acids to Alcohols by the Carboxy-Inversion Reaction«.

Als Persäure zur Bildung des gemischten Persäureanhydrids wird erfindungsgemäß bevorzugt m-Chlorperbenzoesäure eingesetzt. Diese Verbindung steht in technischen Mengen zur Verfügung, ist relativ ungefährlich in der Handhabung und bringt einen glatten Reaktionsablauf. Anstelle von m-Chlorperbenzoesäure können aber auch andere Persäuren eingesetzt werden. Bevorzugt sind dabei aromatische Persäuren, insbesondere solche mit einem elektronenziehenden Rest am aromatischen Kern wie p-Nitroperbenzoesäure.

Die Umsetzung des Steroids-20-Carbonsäurechlorids mit der Persäure, insbesondere mit m-Chlorperbenzoesäure wird bei Temperaturen zwischen −50°C und Raumtemperatur durchgeführt. Die bevorzugten Reaktionstemperaturen liegen zwischen −10 und −30°C. Die Reaktanten werden in etwa stöchiometrischen Mengen eingesetzt. Ein geringer Überschuß der Persäure kann zweckmäßig sein.

Die Umsetzung erfolgt zweckmäßig in einem inerten Lösungsmittel in Gegenwart einer Base. Als Lösungsmittel werden beispielsweise halogenierte Kohlenwasserstoffe wie Methylenchlorid oder Chloroform, Ether wie Diethylether oder Tetrahydrofuran, Nitrile wie Acetonitril, Säureamide wie Dimethylformamid oder Ketone wie z. B. Aceton eingesetzt. Die bevorzugten inerten Verdünnungsmittel sind Lösungsmittel mit einer gewissen Polarität. Als Basen sind tertiäre Stickstoffbasen besonders geeignet. Beispiele hierfür sind Pyridin, Methylpyridine, N,N-Dimethylaminopyridin, Triethylamin oder Ethyldiisopropylamin. Es kommen also sowohl ringförmige als auch offenkettige tertiäre Amine in Betracht. Aber auch anorganische basische Komponenten, insbesondere basische Salze aus starken Basen und schwachen insbesondere flüchtigen Säuren können Verwendung finden. Ein geeignetes Beispiel ist etwa Lithiumcarbonat. Die Base wird vorzugsweise wenigstens in solchen Mengen eingesetzt, daß eine Bindung des bei der Umsetzung entstehenden Halogenwasserstoffs möglich ist.

Die Reaktionsführung erfolgt vorzugsweise derart, daß der Lösung des Steroidcarbonsäurehalogenids im inerten Lösungsmittel nach Kühlung auf die gewünschte Anfangstemperatur der Reaktion — z. B. nach Kühlung auf −30°C — die Persäure und dann anschließend unter Rühren und Kühlen vorsichtig portionsweise die basische Komponente zugesetzt werden. Man läßt dann über einen längeren Zeitraum, z. B. über Nacht auf Raumtemperatur erwärmen und zieht anschließend das Lösungsmittel weitgehend ab.

In der nachfolgenden Reaktionsstufe wird das primär entstandene Umsetzungsprodukt der Verseifung unterworfen. Bevorzugt erfolgt die Verseifung unter basischen Bedingungen und dabei zweckmäßigerweise unter basich/alkoholischen Bedingungen. Geeignet ist zur Verseifung beispielsweise alkoholische Kali- oder Natronlauge. Die Verseifung wird vorzugsweise bei mäßigen Temperaturen, z. B. im Bereich von 0° C vorgenommen. Die basische Komponente wird vorzugsweise im mehrfachen Überschuß über die eingesetzten Säuremengen verwendet. So kann beispielsweise die bei der Verseifung eingesetzte Basenmenge das Drei- bis Achtfache des stöchiometrischen Betrages, bezogen auf eingesetzte Steroidcarbonsäure betragen. Mengen des Vier- bis Sechsfachen an Base sind hier bevorzugt.

Das Verseifungsprodukt wird nach Abschluß der Reaktion eingeengt und dann mit einem inerten mit Wasser nicht mischbaren Lösungsmittel, beispielsweise Methylenchlorid, aufgenommen, gewaschen und dann zur Trockne eingeengt.

Das so erhaltene Rohprodukt ist ein Gemisch der 20-Hydroxy- und der 20-en-Verbindungen. Normalerweise liegt die Hydroxyverbindung in größeren Mengen vor als das 20 (21)-Olefin. Die Komponenten dieses rohen Reaktionsgemisches können beispielsweise durch chromatografische Verfahren voneinander getrennt werden.

Überraschenderweise hat sich gezeigt, daß im Verfahren der Erfindung die neugeschaffene olefinische Bindung stets in 20(21)-Stellung nicht aber — auch nicht teilweise — in der 17(20)-Stellung erscheint.

Bei der Anwendung des Carboxy-Inversions-Abbaus mittels Persäuren auf die erfindungsgemäß eingesetzten BNC-Verbindungen entstehen — je nach der Wahl der eingesetzten Ausgangsverbindung — teils bekannte, teils neue Stoffe als Reaktionsprodukt. Die im Rahmen der Erfindung neu zugänglichen Steroidderivate sind ein weiterer Gegenstand der vorliegenden Erfindung.

Dementsprechend betrifft die Erfindung in einer zusätzlichen Ausführungsform neue $\Delta$20(21)-C-21-Steroidverbindungen mit einer Sauerstoff-Funktion in 11-Stellung und der allgemeinen Formel II

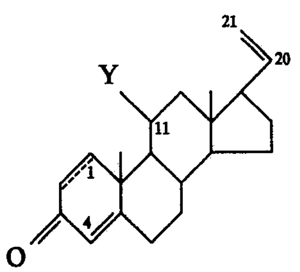

(II)

in der Y eine Hydroxylgruppe oder gemeinsam mit dem durch Y substituierten C-Atom eine Carbonylgruppe bedeutet.

$\Delta$4- bzw. $\Delta$1,4-Verbindungen dieser Art, die eine zusätzliche olefinische Doppelbindung in 20(21)-Stellung und dazu in 11-Stellung eine der angegebenen Sauerstoff-Funktionen aufweisen, sind bisher nicht bekannt. Insbesondere betrifft damit die Erfindung als neue Verbindungen Pregna-1,4,20-trien-3,11-dion und Pregna-4, 20-dien-3,11-dion. In den Rahmen der Erfindung fallen weiterhin die entsprechenden Derivate mit einer 11-Hydroxygruppe, nämlich 11-Hydroxy-pregna-1,4,20-trien-3-on und 11-Hydroxy-pregna-4,20-dien-3-on. Diese Hydroxyverbindungen können die 11-Hydroxygruppe in $\alpha$ oder in $\beta$- Form aufweisen. Die $\beta$-Form kann für pharmakologische Zwecke bevorzugt sein, wobei aber wie eingangs angegeben eine Umwandlung der $\alpha$- in die $\beta$-Form möglich ist. Möglich ist auch die Umwandlung der 11-Hydroxyverbindungen in entsprechende 11-Ketoverbindungen durch Oxidation.

Die neuen Steroid-Alkene der allgemeinen Formel I sind damit insbesondere als Zwischenstufe für die Synthese von Steroidhormonen mit einer Sauerstoff-Funktion an C-11 von Interesse.

Der erfindungsgemäß vorgeschlagene Carboxy-Inversions-Abbau liefert bei den Reaktionsprodukten mit einer Hydroxylgruppe in 20-Stellung Verbindungen, die zum großen Teil bekannt sind. Auch in dieser Unterklasse der erfindungsgemäß erhaltenen Verfahrensprodukte sind jedoch bisher nicht beschriebene Vertreter zu benennen, die in den Rahmen der Erfindung fallen. Hier ist zunächst das 20-Hydroxy-pregna-1,4-dien-3,11-dion bedeutungsvoll, d. h. eine Steroidverbindung, in der in 11-Stellung eine Carbonylgruppe vorliegt. Die Hydroxygruppe in 20-Stellung kann sowohl in $\alpha$- als auch in $\beta$-Konfiguration vorliegen. Als unmittelbare Verfahrensprodukte können erfindungsgemäß die 20-$\alpha$-Verbindungen erhalten werden. Eine weitere neue und erfindungsgemäße Verbindung ist das 11-Hydroxy-20-$\alpha$-hydroxy-pregna-1,4-dien-3-on, wobei hier die Hydroxygruppe in 11-Stellung sowohl in $\alpha$- wie in $\beta$-Form vorliegen kann. Es gelten hier die zuvor gemachten Aussagen für Verbindungen dieses Typs.

Aus den neuen C-20-Alkoholen der angegebenen Strukturen, aber auch aus den erfindungsgemäß herzustellenden, an sich bekannten C-20-Alkoholen lassen sich durch in der organischen Chemie übliche Reaktionen Steroidderivate vom Progesterontyp darstellen.

## Beispiel 1

### Abbau von $\Delta$1,4-BNC zu 20-Hydroxy-pregna-1,4-dien-3-on (A) und Pregna-1,4,20(21)-trien-3-on (B)

Zunächst wird $\Delta$1,4-BNC in das entsprechende Säurechlorid umgewandelt:

17 g (50 mmol) $\Delta$1,4-BNC in 100 ml abs. $CH_2Cl_2$ werden bei 0°C mit 4,0 ml (55 mmol) frisch über Squalen destilliertem Thionylchlorid versetzt und 20 min bei 0°C gerührt. Danach werden das Lösungsmittel und überschüssiges Thionylchlorid bei der gleichen Temperatur im Vakuum entfernt. Der Rückstand wird wieder in Methylenchlorid aufgenommen und die Lösung nochmals zur Trockne eingeengt. Der Rückstand ist für weitere Umsetzungen zu verwenden.

Um ein zur Analyse geeignetes Säurechlorid zu erhalten, wird das rohe Säurechlorid mit abs. Ether digeriert und nach dem Abziehen des Ethers sorgfältig an die Ölpumpe getrocknet.

Das Säurechlorid wird in 100 mlabs. $CH_2Cl_2$ gelöst, dann tropft man bei —40°C zunächst eine Lösung von 10,0 g m-Chlorperbenzoesäure (90%ig, Rest m-Chlorbenzoesäure) in 100 ml abs. Methylenchlorid, danach eine Lösung von 60 mmol trockenem Pyridin in wenig abs. $CH_2Cl_2$ zu und läßt die Mischung über Nacht auf Raumtemperatur kommen.

Das Lösungsmittel wird im Vakuum weitgehend entfernt, der Rückstand wird mit 125 ml 2 n methanolischer Kalilauge versetzt und mehrere Stunden bei Raumtemperatur gerührt. Man zieht einen Teil des Methanols im Vakuum ab, nimmt in Methylenchlorid auf und wäscht die Methylenchlorid-Phase nacheinander mit Wasser, verdünnter Schwefelsäure und wieder mit Wasser.

Nach Trocknen der organischen Phase und Abziehen des Lösungsmittels erhält man 14,6 g kristallinen Rückstand mit 60% (A) und 30% (B). (A) und (B) können durch Chromatografieren auf einer Kieselgelsäule voneinander getrennt werden.

Schmelzpunkt des 20-$\alpha$-Hydroxy-pregna-1,4-dien-3-on (A): 161—166°C.

Schmelzpunkt des Pregna-1,4,20(21)-trien-3-on (B): 170—171°C/Lit.: 166—167°C in J. C. S. Perkin I, 2064 (1979).

MS, IR und NMR-Spektren bestätigen die hier angegebenen Strukturen der Reaktionsprodukte.

Überraschend ist, daß beim Abbau des Säurechlorids neben dem Alkohol (A) selektiv nur das Alken (B) mit 20(21)-Doppelbindung entsteht. Das im Prinzip auch mögliche Alken mit 17(20) -Doppelbindung konnte nicht gefunden werden.

## Beispiel 2

### 20$\alpha$-Hydroxy-pregna-1,4-dien-3,11-dion und Pregna-1,4,20-trien-3,11-dion

a) Pregna-1,4-dien-3,11-dion-20-carbonylchlorid
500 mg (1,4 mmol) $\Delta$1,4-11-Oxo-BNC in 8 ml trockenem $CH_2Cl_2$ werden bei 0°C mit 0,12 ml (1,6 mmol) frisch über Squalen destilliertem Thionylchlorid und einem kleinen Tropfen Pyridin versetzt. Nach 1 h wurde das Lösungsmittel und überschüssiges Thionylchlorid bei 0°C im Vakuum entfernt. Der Rückstand wird mit 5 ml abs. Methylenchlorid versetzt und nochmals zur Trockne eingeengt. Die Auswaage des gebildeten rohen Säurechlorids beträgt 495 mg.

b) Zu der Lösung des Säurechlorids in 5 ml absolutem Methylenchlorid werden bei —30°C 300 mg getrocknete 85%ige m-Chlorperbenzoesäure in 5 ml Methylenchlorid und danach 0,14 ml Pyridin in 2 ml $CH_2Cl_2$ getropft. Man läßt über Nacht auf Raumtemperatur erwärmen, zieht dann das Lösungsmittel (weitgehend) ab und versetzt den Rückstand bei 0°C mit methanolischer Kalilauge (7 mmol KOH). Nach ca. 6 h bei Raumtemperatur engt man ein, nimmt in Methylenchlorid auf und wäscht die Methylenchloridphase nacheinander 2 × mit Wasser, mit verdünnter Schwefelsäure und wieder mit Wasser, trocknet über $Na_2SO_4$ und engt dann zur Trockne ein.
Rohausbeute: 365 mg.
Die säulenchromatografische Trennung (Kieselgel, Methylenchlorid/Essigester 90 : 10 bis 70 : 30) ergab 230 mg (50%) der 20$\alpha$-Hydroxyverbindung und 100 mg (23%) des Alkens in dünnschichtchromatografischer Reinheit. Zur Schmelzpunktbestimmung und für die Elementaranalyse wurden Proben durch präparative Hochdruckflüssigchromatografie fein gereinigt und aus Isopropanol umkristallisiert.

20$\alpha$-OH: FP 237—239 (zers.)°C; $C_{21}H_{28}O_3$
gef. C 76,34%; H 8,43%
ber. C 76,79%; H 8,59%
20(21)-en: Fp 115—127°C; $C_{21}H_{26}O_2$
gef. C 81,02%; H 8,16%
ber. C 81,25%; H 8,44%

Beide Strukturen wurden durch MS, IR und $^1$H-NMR bestätigt.

Beispiel 3

11-$\beta$, 20$\alpha$-Dihydroxy-pregna-1,4-dien-3-on
und 11-$\beta$-Hydroxy-pregna-1,4,20-trien-3-on

a) 11-$\beta$-Hydroxy-pregna-1,4-dien-3-on-20-carbonylchlorid
Die Darstellung wurde analog Beispiels 2a durchgeführt. Zur Überprüfung der Ausbeute wurde das rohe Reaktionsprodukt in den Methylester überführt.
Ausbeute: 88%.

b) Der Carboxy-Inversions-Abbau wurde analog Beispiel 2b durchgeführt. Aus 500 mg der 11-$\beta$-Hydroxy-Säure wurden 218 mg (47%) 20$\alpha$-OH-Verbindung und 122 mg (28%) 20(21)-Alken rein isoliert (Feinreinigung wie in Beispiel 2b angegeben).

20$\alpha$-OH: Fp 210—216° C; $C_{21}H_{30}O_3$
gef. C 76,59%; H 9,31%
ber.C 76,32%; H 9,15%
20(21)-en: Fp 217—222° C; $C_{21}H_{28}O_2$
gef. C 80,61%; H 9,25%
ber. C 80,73%; H 9,03%

Die Strukturen wurden durch MS, IR und $^1$H-NMR bestätigt.

Beispiel 4

Abbau von $\Delta^4$-BNC zu 20-Hydroxy-pregna-4-en-3-on und
Pregna-4,20(21)-dien-3-on

Zu 8,5 g (25 mmol) $\Delta^4$-BNC in 100 ml trockenem $CH_2Cl_2$ werden bei 0° C 3 Tropfen Pyridin und 1,03 ml (14,2 mmol) frisch über Squalen destilliertes Thionylchlorid gegeben. Man rührt 1 Stunde bei 0° C und gibt dann noch einmal die gleiche Menge $SOCl_2$ hinzu.

Nach einer weiteren Stunde werden $CH_2Cl_2$ und überschüssiges Thionylchlorid im Vakuum entfernt. Der Rückstand wird in wenig $CH_2Cl_2$ aufgenommen und nochmals zur Trockne eingeengt.

Die weitere Umsetzung und Aufarbeitung erfolgt so, wie es im Beispiel 1 für den Abbau von $\Delta^{1,4}$-BNC beschrieben wurde. Man erhält 6,1 g kristallinen Rückstand. Durch Chromatografie auf Kieselgel wurden 3,3 g 20-Hydroxy-pregna-4-en-3-on (Fp 163—166° C) und 1,8 g Pregna-4,20(21)-dien-3-on (Fp 122—124° C) erhalten. Die Strukturen wurden durch MS-, IR- und NMR-Spektren bestätigt.

**Patentansprüche**

1. Verfahren zum Abbau der 20-Carboxylgruppe von $\Delta$4- und gegebenenfalls wenigstens eine weitere Doppelbindung in 1(2)- und/oder 9(11)-Stellung aufweisenden Steroid-20-carbonsäuren der allgemeinen Formel I

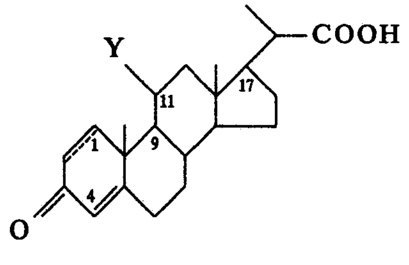

(I)

in der Y Wasserstoff, eine Hydroxylgruppe oder gemeinsam mit dem durch Y substituierten C-Atom eine Carbonylgruppe bedeutet, wobei auch anstelle des Substituenten Y eine 9(11)-en-Bindung vorliegen kann, dadurch gekennzeichnet, daß man die Steroid-20-carbonsäuren über ihre Säurehalogenide vom Carboxy-Inversions-Abbau durch Umsetzung mit Persäuren im Temperaturbereich von —50° C bis Raumtemperatur und Verseifung des Reaktionsproduktes unterwirft und gewünschtenfalls das Verseifungsprodukt in die gebildeten 20-Hydroxy- und die 20(21)-en-Steroidverbindungen auftrennt.

2. Verfahren nach Anspruch 1, dadurch gekennzeichnet, daß man mit m-Chlorperbenzoesäure arbeitet.

3. Verfahren nach Ansprüchen 1 und 2, dadurch gekennzeichnet, daß man die Umsetzung des Steroidcarbonsäurechlorids mit der Persäure bei Temperaturen zwischen —10° C und —30° C, zweckmäßigerweise in einem inerten Lösungsmittel und vorzugsweise in Gegenwart einer basischen Verbindung, insbesondere in Gegenwart von tertiärem Aminen, durchführt.

4. Verfahren nach Ansprüchen 1 bis 3, dadurch gekennzeichnet, daß man die Verseifung des Reaktionsproduktes als basische Alkoholyse durchführt.

5. Neue Δ20(21)-C-21-Steroidverbindungen mit einer Sauerstoff-Funktion in 11-Stellung und der allgemeinen Formel II

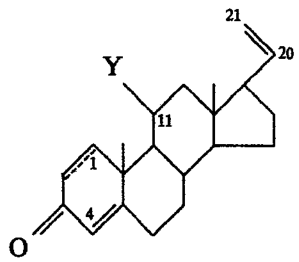

(II)

in der Y eine Hydroxylgruppe oder gemeinsam mit dem durch Y substituierten C-Atom eine Carbonylgruppe bedeutet.

6. 20-Hydroxy-pregna-1,4-dien-3,11-dion.

7. 11-Hydroxy-20-α-Hydroxy-pregna-1,4-dien-3-on.

8. Pregna-1,4,20-trien-3,11-dion.

9. 11-Hydroxy-pregna-1,4,20-trien-3-on.

**Claims**

1. A process for degrading the 20-carboxyl group of Δ4-steroid-20-carboxylic acids optionally containing at least one other double bond in the 1(2)- and/or 9(11)-position and corresponding to the following general formula

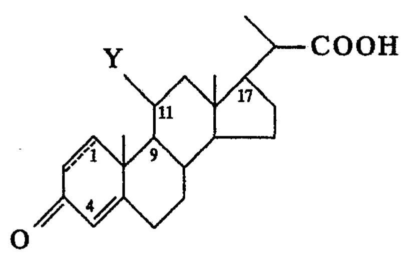

(I)

in which Y represents hydrogen, a hydroxyl group or, together with the C-atom substituted by Y, a carbonyl group, a 9(11)ene bond optionally being present instead of the substituent Y, characterized in that the steroid-20-carboxylic acids are subjected via their acid halides to carboxy inversion degradation by reaction with per acids at temperatures in the range from —50°C room temperature an hydrolysis of the reaction product and, if desired, the hydrolysis product is separated into the 20-hydroxy- and the 20(21)-ene steroid compounds formed.

2. A process as claimed in claim 1, characterized in that m-chloroperbenzoic acid is used.

3. A process as claimed in claims 1 and 2, characterized in that the reaction of the steroid carboxylic acid chloride with the per acid is carried out at temperatures of from —10°C to —30°C, best in an inert solvent and preferably in the presence of a basic compound, more particularly in the presence of tertiary amines.

4. Process as claimed in claims 1 to 3, characterized in that hydrolysis of the reaction product is carried out as basic alcoholysis.

5. New Δ20(21)-C-21-steroid compounds containing an oxygen function in the 11-position and corresponding to the following general formula

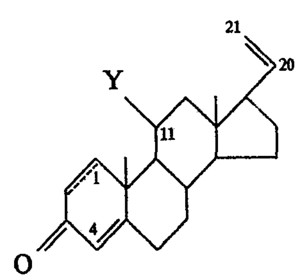

(II)

in which Y is a hydroxyl group or, together with the C-atom substituted by Y, represents a carbonyl group.

6. 20-Hydroxy-pregna-1,4-dien-3,11-dione.
7. 11-Hydroxy-20-α-hydroxy-pregna-1,4-dien-3-one.
8. Pregna-1,4,20-trien-3,11-dione.
9. 11-Hydroxy-pregna-1-4,20-trien-3-one.

## Revendications

1. Procédé pour la dégradation du groupe 20-carboxyle dans des acides stéroïde-20-carboxyliques à double liaison Δ4- et le cas échéant au moins une autre double liaison en position 1(2) et/ou 9(11) de formule générale I

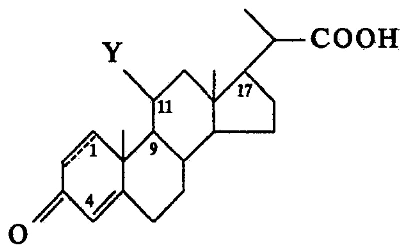

(I)

dans laquelle Y représente l'hydrogène, un groupe hydroxy ou forme en commun avec l'atome de carbone substitué par Y un groupe carbonyle, le substituant Y pouvant également être remplacé par une liaison 9(11)-ène, caractérisé en ce que l'on soumet les acides stéroïde-20-carboxyliques, en passant par l'intermédiaire de leurs halogénures d'acides, à la dégradation par inversion du groupe carboxy par réaction avec des peracides dans un intervalle de température de −50° C à la température ambiante et saponification du produit de réaction, et, le cas échéant, on sépare le produit de saponification en les composés en 20-hydroxy et 20(21)-ène formés.

2. Procédé selon la revendication 1, caractérisé en ce que l'on opère avec l'acide m-chloroperbenzoïque.

3. Procédé selon les revendications 1 et 2, caractérisé en ce que la réaction du chlorure de l'acide stéroïde-carboxylique avec le peracide est effectuée à des températures allant de −10 à −30° C, de préférence dans un solvant inerte et de préférence en présence d'un composé basique, en particulier en présence d'amines tertiaires.

4. Procédé selon les revendications 1 à 3, caractérisé en ce que la saponification du produit de réaction est réalisée par alcoolyse basique.

5. Nouveaux composés Δ20(21)-C-21-stéroïdiques ayant une fonction oxygénée en position 11 et répondant à la formule générale II

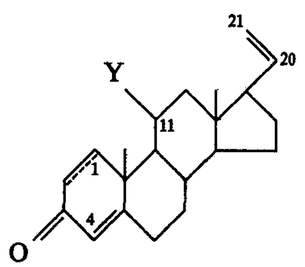

(II)

dans laquelle Y représente un groupe hydroxy ou forme un groupe carbonyle avec l'atome de carbone substitué par Y.

6. La 20-hydroxy-prégna-1,4-diène-3,11-dione.
7. La 11-hydroxy-20α-hydroxy-prégna-1,4-diène-3-one.
8. La prégna-1,4,20-triène-3,11-dione.
9. La 11-hydroxy-prégna-1,4,20-triène-3-one.